(19) **Europäisches Patentamt / European Patent Office / Office européen des brevets**

(11) **EP 4 276 442 B1**

(12) **EUROPEAN PATENT SPECIFICATION**

(45) Date of publication and mention
of the grant of the patent:
**13.05.2026 Bulletin 2026/20**

(21) Application number: **22795178.7**

(22) Date of filing: **18.01.2022**

(51) International Patent Classification (IPC):
**G01N 17/00** (2006.01)  **G01N 3/00** (2006.01)
**G01N 3/20** (2006.01)  G01N 33/2045 (2019.01)

(52) Cooperative Patent Classification (CPC):
**G01N 3/20; G01N 17/006; G01N 33/2045;**
G01N 2203/0064; G01N 2203/0066;
G01N 2203/024; G01N 2203/0274;
G01N 2203/0282; G01N 2203/0298;
G01N 2203/0647; G01N 2203/0682

(86) International application number:
**PCT/JP2022/001619**

(87) International publication number:
**WO 2022/230255 (03.11.2022 Gazette 2022/44)**

(54) **METHOD FOR TESTING SULFIDE STRESS CORROSION CRACKING OF STEEL MATERIAL**

VERFAHREN ZUM TESTEN VON SULFIDSPANNUNGSRISSKORROSION VON STAHLMATERIAL

PROCÉDÉ DESTINÉ À ÉPROUVER LA FISSURATION DE CORROSION SOUS CONTRAINTE DE SULFURE DE MATÉRIAU D'ACIER

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.04.2021 JP 2021077743**

(43) Date of publication of application:
**15.11.2023 Bulletin 2023/46**

(73) Proprietor: **JFE Steel Corporation
Tokyo 100-0011 (JP)**

(72) Inventors:
• **SAKIMOTO Takahiro
  Tokyo 100-0011 (JP)**
• **HANDA Tsunehisa
  Tokyo 100-0011 (JP)**

(74) Representative: **Grünecker Patent- und
Rechtsanwälte
PartG mbB
Leopoldstraße 4
80802 München (DE)**

(56) References cited:
WO-A1-2012/121106  CN-A- 103 207 141
CN-A- 112 577 822  JP-A- 2006 010 427
JP-A- 2008 051 632  JP-A- 2008 051 632
JP-A- 2013 019 842  JP-A- H1 123 565
US-A1- 2008 216 928

• SHIMAMURA JUNJI, DAICHI IZUMI, ITARU SAMUSAWA, SATOSHI IGI: "Effect of Surface Hardness and Hydrogen Sulfide Partial Pressure on Sulfide Stress Cracking Behavior in Low Alloy Linepipe Steel", TETSU TO HAGANE: JOURNAL OF THE IRON AND STEEL INSTITUTE OF JAPAN, vol. 107, no. 5, 1 January 2021 (2021-01-01), JP , pages 356 - 366, XP055981564, ISSN: 0021-1575, DOI: 10.2355/tetsutohagane.TETSU-2020-121

## Description

Technical Field

The present invention relates to a method for testing sulfide stress corrosion cracking of a steel material such as a steel pipes for line pipes.

Background Art

Fluids produced from deep-water oil fields or gas fields, which have been being developed in recent years, often contain corrosive hydrogen sulfide. In such an environment, high-strength steel comes to break due to the occurrence of hydrogen embrittlement called sulfide stress cracking (hereinafter, referred to as SSC). As the development of deep-water oil fields or gas fields progresses, an SSC resistance of steel pipes for line pipes in use has become important. In the past, there was a problem of gas leakage attributed to SSC immediately after pipeline operation or the like, and there has been a demand for a test technology for accurately evaluating the SSC resistance of materials in use from the viewpoint of safety.

Ordinarily, the SSC resistance of materials is evaluated by a double cantilever beam (DCB) test according to a method D specified in National Association of Corrosion Engineers (NACE) Standard TM0177-2005 of NPL 1. In addition, a stress intensity factor $K_{ISSC}$ value under a sulfide corrosion environment is calculated from the measured value of this DCB test. The stress intensity factor $K_{ISSC}$ value that is obtained by the DCB test is an index indicating the lowest K value at which cracks are capable of propagating under a given corrosion environment (the intensity of a stress field at a crack tip portion), and it means that, as the larger this value, the lower the cracking susceptibility in the given corrosion environment.

Citation List

Non Patent Literature

NPL 1: NACE Standard TM0177-2005

Relevant prior art can be found in document JP2008051632 which describes a stress-corrosion-cracking progress test-device method wherein a test piece having an initial crack is immersed in a test solution in a 4-point bending test.

Summary of Invention

Technical Problem

By the way, in the DCB test according to the method D specified in NPL 1, it is necessary to collect a test piece from a steel pipe or a steel sheet in a direction in which a sheet thickness of the steel pipe or the steel sheet matches the thickness of the test piece due to dimensional restrictions, and a crack introduction direction of the test piece at that time is a surface direction perpendicular to a sheet thickness direction.

However, since SSC in a steel pipe for line pipes occurs from the surface of the material, an actual crack propagation direction is the sheet thickness direction. Ordinarily, steel pipes manufactured by rolling have different microstructure distribution and strength and toughness characteristics in a sheet thickness direction and in a sheet thickness perpendicular direction due to microstructure control by rolling. Therefore, the $K_{ISSC}$ value obtained by the DCB test cannot be said to represents steel sheet characteristics in a direction in which actual SSC occurs, and, in actual environments, there is a risk of the occurrence of unexpected SSC, which may lead to serious accidents. Due to such a fact, there has been a strong demand for a method for obtaining the $K_{ISSC}$ value of a material in a direction in which actual SSC occurs.

Therefore, the present invention has been made to solve this conventional problem, and an object of the present invention is to provide a method for testing sulfide stress corrosion cracking of a steel material enabling the obtainment of the stress intensity factor $K_{ISSC}$ value of a steel material to be evaluated in the sheet thickness direction.

Solution to Problem

In order to solve the above-described problem, a method for testing sulfide stress corrosion cracking of a steel material according to the present invention is as specified in claim 1.

Advantageous Effects of Invention

According to the method for testing sulfide stress corrosion cracking of a steel material according to the present invention, it is possible to provide a method for testing sulfide stress corrosion cracking of a steel material enabling the obtainment of a stress intensity factor $K_{ISSC}$ value in the sheet thickness direction of a steel material to be evaluated. In addition, the same aspect can also be applied to a method for testing ammonia stress corrosion cracking in tanks and vessels for ammonia, not part of the claimed invention.

Brief Description of Drawings

FIG. 1 is a flowchart for describing the flow of a treatment in a method for testing sulfide stress corrosion cracking of a steel material according to an embodiment of the present invention;
FIG. 2 is a flowchart for describing the details of a step S5 (stress intensity factor calculation step) in the flowchart of FIG. 1;
FIG. 3 is a perspective view of a state where a plurality of initial cracks has been introduced into a test piece to be tested by the method for testing sulfide stress corrosion cracking;
FIG. 4 is a cross-sectional view for describing dimensions of an initial crack introduced into the test piece illustrated in FIG. 3;
FIG. 5 is a view for describing a 4-point bending SSC test jig according to NACE TM0316-2016;
FIG. 6 is a perspective view of an example of a test piece on which a 4-point bending SSC test has been performed using the 4-point bending test jig illustrated in FIG. 5; and
FIG. 7 is a view for describing an example.

Description of Embodiments

Hereinafter, an embodiment of the present invention will be described with reference to drawings. The embodiment to be described below exemplifies devices or methods for embodying a technical idea of the present invention, and the technical idea of the present invention does not specify the material, shape, structure, arrangement, and the like of a configuration component to the following embodiment. In addition, the drawings are schematic. Therefore, it should be noted that a relationship, ratio, and the like between the thickness and a plane dimension are different from the actual ones, and there are parts where the relationship or ratio of the dimensions are different between the drawings.

The present inventors studied a variety of methods regarding a method for testing sulfide stress corrosion cracking by which the stress intensity factor $K_{ISSC}$ value in the sheet thickness direction of a steel material to be evaluated is obtained.

In ordinary 4-point bending SSC test methods, there are no initial cracks in a test piece, and whether or not SSC occurs and propagates from the surface of the material is determined, and it is not possible to measure a crack length or a stress intensity factor $K_{ISSC}$ value at which rupture by SSC occurs.

Therefore, the present inventors newly found the following phenomenon and invented a method for testing sulfide stress corrosion cracking for obtaining the stress intensity factor $K_{ISSC}$ value in the sheet thickness direction of a steel material to be evaluated.

A 4-point bending SSC test is performed by introducing, into a flat plate-shaped test piece that is collected from a steel material to be evaluated, a plurality of fine initial cracks that extends in the sheet thickness direction from the surface of the test piece and has the same length (2 $\mu$m to 1000 $\mu$m in width and 1 $\mu$m to 1000 $\mu$m in length) and immersing the test piece into which the plurality of fine initial cracks has been introduced in a test solution in a 4-point bent state. Then, the crack gradually propagates from each initial crack by SSC during the test, and, when each of the propagated cracks reaches a specific crack length, the test piece abruptly comes to rupture. At that time, at each of the cracks that have propagated at almost the same rate, the load applied to the test piece is unloaded when the test piece has ruptured from a certain crack, and the cracks do not propagate any longer in the other cracks. In addition, the lengths of the remaining cracks and the applied load (bending stress with respect to the test piece) are measured, whereby it is possible to obtain the stress intensity factor $K_{ISSC}$ value immediately before the test piece ruptures due to SSC.

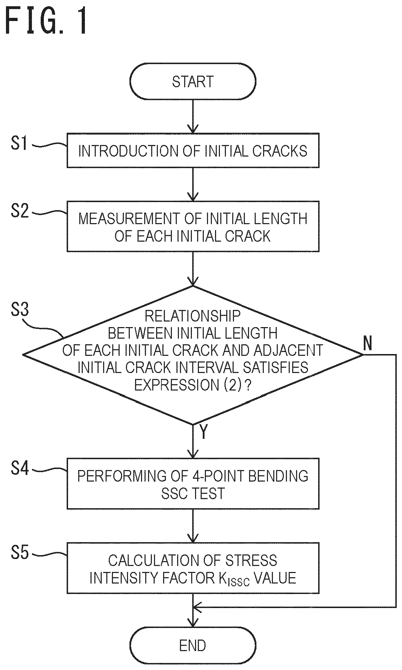

FIG. 1 illustrates a flowchart for describing the flow of a treatment in a method for testing sulfide stress corrosion cracking of a steel material according to an embodiment of the present invention.

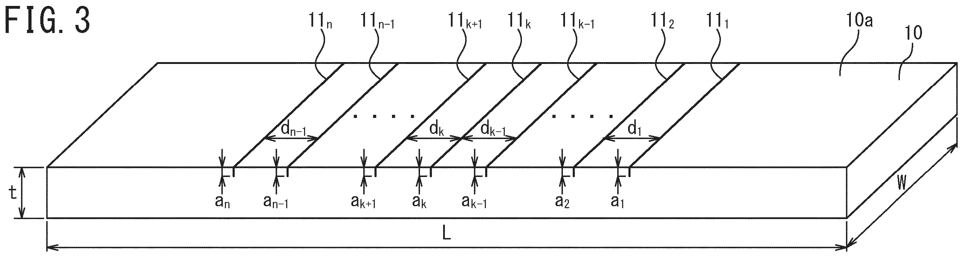

In the method for testing sulfide stress corrosion cracking, first, in a step S1, fine initial cracks $11_1$ to $11_n$ extending in the sheet thickness direction from a surface 10a of a test piece 10 are introduced into the flat plate-shaped test piece 10 (refer to FIG. 3) that is collected from a steel material to be evaluated (crack introduction step).

Specifically, the flat plate rectangular test piece 10 as illustrated in FIG. 3 is collected from a steel material to be evaluated such as a steel pipe for a line pipe. A length L of the test piece 10 is, for example, approximately 125 mm, a width W is, for example, approximately 25 mm, and a sheet thickness t is, for example, approximately 5 mm.

In addition, the plurality (first to $n^{th}$, n = 2 or more) of initial cracks $11_1$ to $11_n$ extending in the sheet thickness direction from the surface 10a of the test piece 10 is introduced into this test piece 10 in a row along a longitudinal direction (row direction) of the test piece 10. The first initial crack $11_1$ to the $n^{th}$ initial crack $11_n$ are formed in order at predetermined intervals $d_1$ to $d_{n-1}$ (for example, approximately 10 mm) along the row direction from the longitudinal-direction right end side (right end side in FIG. 3) toward the longitudinal-direction left end side of a test piece S. $d_1$ is the interval between the (first and second) initial cracks $11_1$:$11_2$ adjacent to each other, $d_2$ is the interval between the (second and third) initial cracks $11_2$:$11_3$ adjacent to each other, ..., $d_{n-1}$ is the interval between the (n-1$^{th}$ and n$^{th}$) initial cracks $11_{n-1}$:$11_n$ adjacent to each other.

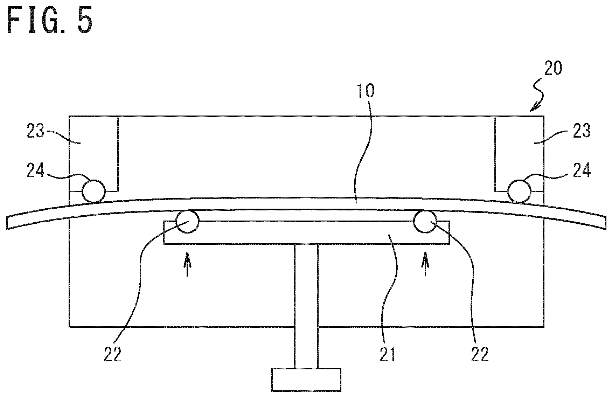

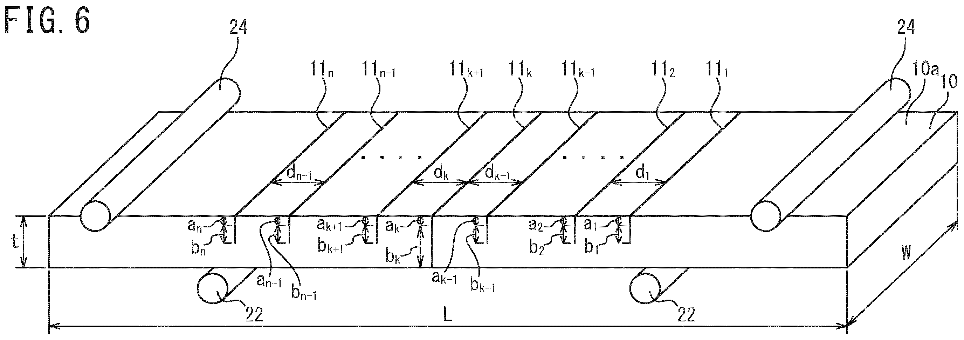

The plurality of fine initial cracks $11_1$ to $11_n$ is introduced into the inside of a 4-point bending load jig 22 of a short span of a 4-point bending SSC test jig 20 (refer to FIG. 5 and FIG. 6), which is a uniform stress region, whereby uniform stress is applied to each of the initial cracks $11_1$ to $11_n$.

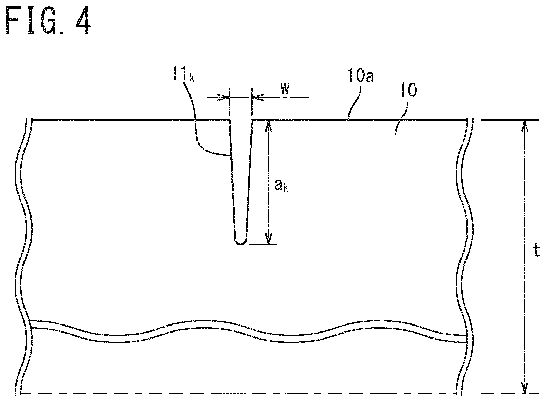

The dimensions of each of the initial cracks $11_1$ to $11_n$ are illustrated in FIG. 4 (only the dimension of the $k^{th}$ initial crack $11_k$ are illustrated in FIG. 4), the dimensions are desirably approximately the same as those of a pit that is observed in an actual corrosion environment or corrosion test, the width w is 2 $\mu$m to 1000 $\mu$m, and the initial lengths (depths) $a_1$ to $a_n$ are 1 $\mu$m to 1000 $\mu$m. For the processing of each of the initial cracks $11_1$ to $11_n$, a method in which a thermal effect during the processing is decreased as much as possible using a short pulse processing machine such as a picosecond laser is desirable, and, when the width w of each of the initial cracks $11_1$ to $11_n$ is less than 2 $\mu$m and the initial lengths $a_1$ to $a_n$ are less than 1 $\mu$m, the processing is difficult. On the other hand, the widths w of the initial cracks $11_1$ to $11_n$ of larger than 1000 $\mu$m or the initial lengths $a_1$ to $a_n$ of larger than 1000 $\mu$m are unrealistic. Regarding the dimensions of each of the initial cracks $11_1$ to $11_n$, the width w of 30 $\mu$m to 150 $\mu$m and the initial lengths (depths) $a_1$ to $a_n$ of 120 $\mu$m to 500 $\mu$m are close to pit dimensions that are shown in actual corrosion tests and particularly preferable. The initial lengths $a_1$ to $a_n$ of each of the initial cracks $11_1$ to $11_n$ are set to be the same. In addition, the intervals $d_1$ to $d_{n-1}$ between (first and second, ..., n-1$^{th}$ and n$^{th}$) initial cracks adjacent to each other $11_1$:$11_2$, ..., $11_{n-1}$:$11_n$ can be set arbitrarily.

In FIG. 3, each of the initial cracks $11_1$ to $11_n$ is introduced over the entire length of the test piece 10 in a width direction, but the lengths of the initial cracks are not limited thereto.

Next, in a step S2, the initial lengths $a_1$ to $a_n$ of each of the plurality (first to n$^{th}$) of initial cracks $11_1$ to $11_n$ introduced in the step S1 are measured (initial length measuring step).

Next, in a step S3, whether or not the relationship between the initial lengths $a_1$ to $a_n$ of each of the plurality (first to n$^{th}$) of measured initial cracks $11_1$ to $11_n$ and the intervals $d_1$ to $d_{n-1}$ between (first and second, ..., n-1$^{th}$ and n$^{th}$) initial cracks adjacent to each other $11_1$:$11_2$, ..., $11_{n-1}$:$11_n$ satisfies Expression (2) below is determined (adjacent crack determination step).

$$d_1/a_1 \geq 5,\ d_1/a_2 \geq 5,\ d_2/a_2 \geq 5,\ ...,\ d_{n-2}/a_{n-1} \geq 5,\ d_{n-1}/a_{n-1} \geq 5,\ \text{and}\ d_{n-1}/a_n \geq 5 \tag{2}$$

Here, $a_1$ to $a_n$ are the initial lengths of each of the plurality (first to n$^{th}$) of initial cracks $11_1$ to $11_n$, and $d_1$ to $d_{n-1}$ are the intervals between (first and second, ..., n-1$^{th}$ and n$^{th}$) initial cracks adjacent to each other $11_1$:$11_2$, ..., $11_{n-1}$:$11_n$.

In order to obtain the stress intensity factor $K_{ISSC}$ value, it is necessary for adjacent initial cracks to propagate under almost the same conditions, and, in order for that, it is necessary to decrease the influence of the stress state at each crack tip on other adjacent cracks as much as possible. Therefore, whether or not the relationship between the initial lengths $a_1$ to $a_n$ of each of the plurality (first to n$^{th}$) of measured initial cracks $11_1$ to $11_n$ and the intervals $d_1$ to $d_{n-1}$ between (first and second, ..., n-1$^{th}$ and n$^{th}$) initial cracks adjacent to each other $11_1$:$11_2$, ..., $11_{n-1}$:$11_n$ satisfies Expression (2) is determined, and, only in a case where Expression (2) is satisfied, the treatment proceeds to the following step S4 (4-point bending SSC test step). A stress field analysis of crack tips was performed on the intervals $d_1$ to $d_{n-1}$ between the initial cracks adjacent to each other $11_1$:$11_2$, ..., $11_{n-1}$:$11_n$ and the initial lengths $a_1$ to $a_n$ of each of the initial cracks $11_1$ to $11_n$, when the intervals $d_1$ to $d_{n-1}$ between the adjacent initial cracks $11_1$:$11_2$, ..., $11_{n-1}$:$11_n$ became narrow, and the initial lengths $a_1$ to $a_n$ of each of the initial cracks $11_1$ to $11_n$ became long, the fact that the influence became large between the initial cracks adjacent to each other $11_1$:$11_2$, ..., $11_{n-1}$:$11_n$ was clarified, and the optimum conditions of Expression (2) were derived.

Next, when the determination result in the step S3 is YES, the treatment proceeds to a step S4, and, when the determination result in the step S3 is No (any one of the initial lengths $a_1$ to $a_n$ of the initial cracks $11_1$ to $11_n$ does not satisfy Expression (2)), the sulfide stress corrosion cracking test is ended.

In addition, in the step S4, a 4-point bending SSC test is performed by immersing the test piece 10 into which the initial cracks $11_1$ to $11_n$ have been introduced in the step S1 (crack introduction step) in a test solution in a 4-point bent state (4-point bending SSC test step).

Here, the 4-point bending SSC test is performed according to NACE TM0316-2016, and a 4-point bending SSC test jig that is used for the 4-point bending SSC test is illustrated in FIG. 5.

The 4-point bending SSC test jig 20 illustrated in FIG. 5 includes a pedestal 21 in which two 4-point bending load jigs 22 are arranged with a short span and support members 23 that are above the two 4-point bending load jigs 22 and are supported with a long span that is longer than the span with which the two 4-point bending load jigs 22 are arranged. In addition, the test piece 10 into which the plurality of initial cracks $11_1$ to $11_n$ have been introduced is sandwiched between the two 4-point bending load jigs 22 arranged in the pedestal 21 and two 4-point bending load jigs 24 supported by the support members 23, and the pedestal 21 is raised to apply a predetermined bending stress to the test piece 10 supported at four points. In this state, the test piece 10 is immersed in the test solution and left to stand in that state for a predetermined period (for example, approximately 30 days).

Next, in a step S5, the stress intensity factor $K_{ISSC}$ value is calculated based on the state where the initial cracks $11_1$ to $11_n$ have propagated in the sheet thickness direction in the step S4 (4-point bending SSC test step) (stress intensity factor calculation step).

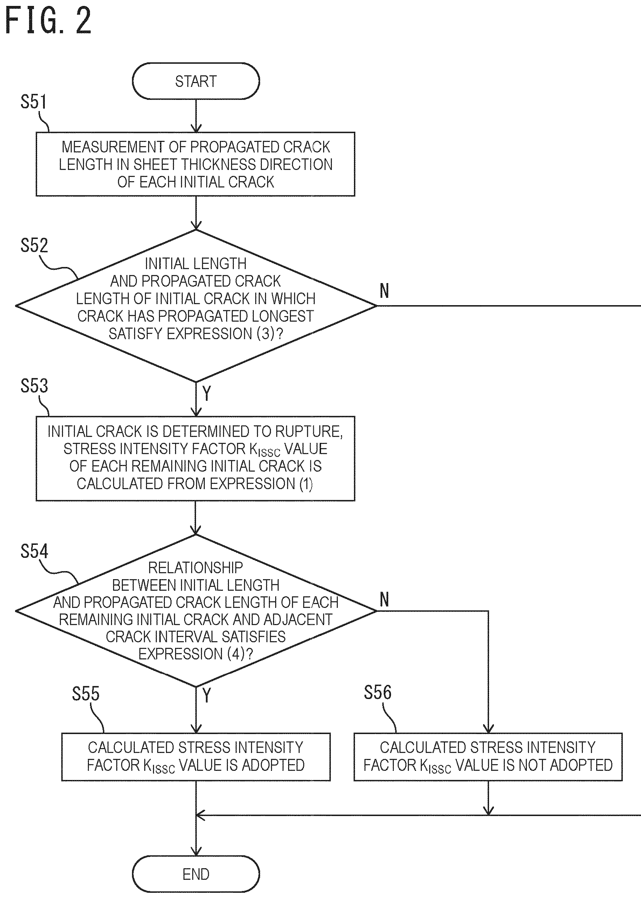

In this stress intensity factor calculation step, as illustrated in FIG. 2, first, in a step S51, propagated crack lengths $b_1$ to $b_n$ in the sheet thickness direction of each of the plurality (first to $n^{th}$) of initial cracks $11_1$ to $11_n$ in the step S4 (4-point bending SSC test step) (refer to FIG. 6) are measured (propagated crack length measurement step).

Next, in a step S52, among the plurality (first to $n^{th}$) of initial cracks $11_1$ to $11_n$, an initial crack that has propagated the longest in the sheet thickness direction is designated as any one ($k^{th}$ among first to $n^{th}$) of initial crack $11_k$ in a target to be ruptured, and whether or not the initial length $a_k$ and propagated crack length $b_k$ of the any one ($k^{th}$ among first to $n^{th}$) of initial crack $11_k$ in the target to be ruptured satisfy Expression (3) below is determined (rupture condition determination step).

$$0.8t \leq (a_k + b_k) \leq 1.0t \ ... \ (3)$$

Here, $a_k$ is the measured initial length of the any one ($k^{th}$ among first to $n^{th}$) of initial crack $11_k$ in the target to be ruptured, $b_k$ is the measured propagated crack length of the any one ($k^{th}$ among first to $n^{th}$) of initial crack $11_k$ in the target to be ruptured, and t is the sheet thickness of a test piece.

The reason for designating, among this plurality (first to $n^{th}$) of initial cracks $11_1$ to $11_n$, the initial crack that has propagated the longest in the sheet thickness direction as the any one ($k^{th}$ among first to $n^{th}$) of initial crack $11_k$ in the target to be ruptured and a point in time where the initial length $a_k$ and propagated crack length $b_k$ of the any one ($k^{th}$ among first to $n^{th}$) of initial crack $11_k$ in the target to be ruptured have exceeded 80% of the sheet thickness t of the test piece 10 is as described below. That is, it is because it was found from the calculation of a stress field by a finite element analysis in which the 4-point bending SSC test was reproduced that, when the propagated cracks exceed 80% of the sheet thickness t of the test piece 10, the status is the same as when a 4-point bending load does not act on the remaining cracks any longer.

In addition, when the determination result in the step 52 is YES (when Expression (3) below is satisfied), the treatment proceeds to a step S53, and, when the determination result is NO, the stress intensity factor calculation step is ended, and the sulfide stress corrosion cracking test is ended.

In addition, in the step S53, the any one initial crack $11_k$ ($k^{th}$ among first to $n^{th}$) in the target to be ruptured has been determined to rupture, and the stress intensity factor $K_{ISSC}$ values of a plurality (first to k-$1^{th}$ and k+$1^{th}$ to $n^{th}$) of respective remaining initial cracks $11_1$ to $11_{k-1}$ and $11_{k+1}$ to $11_n$ are calculated from Expression (1) below based on the initial lengths $a_1$ to $a_{k-1}$ and $a_{k+1}$ to $a_n$ and propagated crack lengths $b_1$ to $b_{k-1}$ and $b_{k+1}$ to $b_n$ of the remaining initial cracks $11_1$ to $11_{k-1}$ and $11_{k+1}$ to $11_n$ (calculation step) .

[Math. 1]

$$K_{ISSC1} = F \cdot \sigma \sqrt{\pi(a_1 + b_1)} \ , \ K_{ISSC2} = F \cdot \sigma \sqrt{\pi(a_2 + b_2)} \ , \cdots ,$$

$$K_{ISSCk-1} = F \cdot \sigma \sqrt{\pi(a_{k-1} + b_{k-1})} \ , \ K_{ISSCk+1} = F \cdot \sigma \sqrt{\pi(a_{k+1} + b_{k+1})} \ , \cdots ,$$

$$K_{ISSCn} = F \cdot \sigma \sqrt{\pi(a_n + b_n)} \ \cdots \ (1)$$

Here, $K_{ISSC1}$ to $K_{ISSCk-1}$ and $K_{ISSCk+1}$ to $K_{ISSCn}$ are the stress intensity factor $K_{ISSC}$ values of the respective remaining initial cracks $11_1$ to $11_{k-1}$ and $11_{k+1}$ to $11_n$, F is the shape factor, $\sigma$ is a bending stress that is applied to the test piece 10 by the 4-point bending SSC test step, $a_1$ to $a_{k-1}$ and $a_{k+1}$ to $a_n$ are the measured initial lengths of the respective remaining initial cracks $11_1$ to $11_{k-1}$ and $11_{k+1}$ to $11_n$, and $b_1$ to $b_{k-1}$ and $b_{k+1}$ to $b_n$ are the measured propagated crack lengths of the respective remaining initial cracks $11_1$ to $11_{k-1}$ and $11_{k+1}$ to $11_n$. The shape factor F is set to 1.2 in the present embodiment.

Next, in a step S54, whether or not the relationship between the initial lengths $a_1$ to $a_{k-1}$ and $a_{k+1}$ to $a_n$ and

propagated crack lengths $b_1$ to $b_{k-1}$ and $b_{k+1}$ to $b_n$ of the respective remaining initial cracks $11_1$ to $11_{k-1}$ and $11_{k+1}$ to $11_n$ and the intervals $d_1$ to $d_{n-1}$ between the cracks adjacent to each other satisfies Expression (4) below is determined (crack length determination step).

$$d_1/(a_1 + b_1) \geq 5, \, d_1/(a_2 + b_2) \geq 5, \, d_2/(a_2 + b_2) \geq 5, \, ..., \, d_{k-2}/(a_{k-1} + b_{k-1}) \geq 5, \, d_{k-1}/(a_{k-1} + b_{k-1}) \geq 5, \, d_k/(a_{k+1} + b_{k+1}) \geq 5, \, d_{k+1}/(a_{k+1} + b_{k+1}) \geq 5, \, ..., \, d_{n-1}/(a_{n-1} + b_{n-1}) \geq 5, \, d_{n-1}/(a_n + b_n) \geq 5 \tag{4}$$

Here, $a_1$ to $a_{k-1}$ and $a_{k+1}$ to $a_n$ are the measured initial lengths of the respective remaining initial cracks $11_1$ to $11_{k-1}$ and $11_{k+1}$ to $11_n$, $b_1$ to $b_{k-1}$ and $b_{k+1}$ to $b_n$ are the measured propagated crack lengths of the respective remaining initial cracks $11_1$ to $11_{k-1}$ and $11_{k+1}$ to $11_n$, and $d_1$ to $d_{n-1}$ are the intervals between the cracks $11_1{:}11_2$, ..., $11_{n-1}{:}11_n$ adjacent to each other.

In order to obtain the stress intensity factor $K_{ISSC}$ value, it is necessary for adjacent initial cracks to propagate under almost the same conditions, and, in order for that, it is necessary to decrease the influence of the stress state at each crack tip on other adjacent cracks as much as possible. Therefore, when one crack (initial crack $11_k$) has ruptured, whether or not the crack lengths (the initial lengths $a_1$ to $a_{k-1}$ and $a_{k+1}$ to $a_n$ + the propagated crack lengths $b_1$ to $b_{k-1}$ and $b_{k+1}$ to $b_n$) of the remaining initial cracks $11_1$ to $11_{k-1}$ and $11_{k+1}$ to $11_n$ that have propagated until immediately before the rupture satisfy Expression (4), and, only in a case where the expression is satisfied, the stress intensity factors $K_{ISSC}$ values of the respective remaining initial cracks $11_1$ to $11_{k-1}$ and $11_{k+1}$ to $11_n$ calculated in the step S53 are adopted. A stress field analysis of the crack tips was performed on the intervals $d_1$ to $d_{n-1}$ between the initial cracks adjacent to each other $11_1{:}11_2$, ..., $11_{n-1}{:}11_n$ and the crack lengths (the initial lengths $a_1$ to $a_{k-1}$ and $a_{k+1}$ to $a_n$ + the propagated crack lengths $b_1$ to $b_{k-1}$ and $b_{k+1}$ to $b_n$) of the remaining initial cracks $11_1$ to $11_n$, when the intervals $d_1$ to $d_{n-1}$ between the adjacent initial cracks $11_1{:}11_2$, ..., $11_{n-1}{:}11_k$ were narrow, and the crack lengths (the initial lengths $a_1$ to $a_{k-1}$ and $a_{k+1}$ to $a_n$ + the propagated crack lengths $b_1$ to $b_{k-1}$ and $b_{k+1}$ to $b_n$) of the remaining initial cracks $11_1$ to $11_{k-1}$ and $11_{k+1}$ to $11_n$ became long, the fact that the influence became large between the initial cracks adjacent to each other $11_1{:}11_2$, ..., $11_{n-1}{:}11_n$ was clarified, and the optimum conditions of Expression (4) were derived.

In addition, when the determination result in the step S54 is YES (when Expression (4) is satisfied), the treatment proceeds to a step S55, the stress intensity factors $K_{ISSC}$ values of the respective remaining initial cracks $11_1$ to $11_{k-1}$ and $11_{k+1}$ to $11_n$ calculated in the step S53 are adopted, the stress intensity factor calculation step is ended, and the sulfide stress corrosion cracking test is ended.

On the other hand, when the determination result in the step S54 is NO (when Expression (4) is not satisfied), the treatment proceeds to a step S56, the stress intensity factors $K_{ISSC}$ values of the respective remaining initial cracks $11_1$ to $11_{k-1}$ and $11_{k+1}$ to $11_n$ calculated in the step S53 are not adopted, the stress intensity factor calculation step is ended, and the sulfide stress corrosion cracking test is ended.

As described above, according to the method for testing sulfide stress corrosion cracking of a steel material according to the present embodiment, a crack introduction step (step S1) of introducing, into the flat plate-shaped test piece 10 that is collected from a steel material to be evaluated, the initial cracks $11_1$ to $11_n$ that extends in the sheet thickness direction from the surface 10a of the test piece 10 and is 2 $\mu$m to 1000 $\mu$m in width w and 1 $\mu$m to 1000 $\mu$m in initial lengths $a_1$ to $a_n$, a 4-point bending SSC test step (step S4) of performing the 4-point bending SSC test in which the test piece 10 into which the initial cracks $11_1$ to $11_n$ have been introduced in the crack introduction step (step S1) is immersed in the test solution in a 4-point bent state and a stress intensity factor calculation step (step S5) of calculating the stress intensity factor $K_{ISSC}$ values based on a state where the initial cracks $11_1$ to $11_n$ have propagated in the sheet thickness direction by the 4-point bending SSC test step (step S4) are included.

Therefore, it is possible to obtain the stress intensity factor $K_{ISSC}$ value in the sheet thickness direction of the steel material to be evaluated.

In addition, according to the method for testing sulfide stress corrosion cracking of a steel material according to the present embodiment, in the crack introduction step (step S1), the plurality (first to $n^{th}$, n = 2 or more) of initial cracks $11_1$ to $11_n$ that extends in the sheet thickness direction from the surface 10a of the test piece 10 is introduced along the row direction, and, in the 4-point bending SSC test step (step S4), the test piece 10 into which the plurality (first to $n^{th}$) of initial cracks $11_1$ to $11_n$ has been introduced is immersed in the test solution in a 4-point bent state. In addition, in the stress intensity factor calculation step (step S5), the stress intensity factors $K_{ISSC}$ values of the respective remaining initial cracks $11_1$ to $11_{k-1}$ and $11_{k+1}$ to $11_n$ are calculated from Expression (1) based on the initial lengths $a_1$ to $a_{k-1}$ and $a_{k+1}$ to $a_n$ and propagated crack lengths $b_1$ to $b_{k-1}$ and $b_{k+1}$ to $b_n$ of the plurality (first to $k-1^{th}$ and $k+1^{th}$ to $n^{th}$) of initial cracks $11_1$ to $11_{k-1}$ and $11_{k+1}$ to $11_n$ remaining in the sample ruptured due to the propagation in the sheet thickness direction of the any one initial crack $11_k$ ($k^{th}$ among first to $n^{th}$) among the plurality (first to $n^{th}$) of initial cracks $11_1$ to $11_n$ by the 4-point bending SSC test step (step S4).

Therefore, based on a phenomenon in which the 4-point bending SSC test is performed by immersing the test piece 10 into which the plurality of fine initial cracks $11_1$ to $11_n$ has been introduced in the test solution in a 4-point bent state, cracks gradually propagate from the respective initial cracks $11_1$ to $11_n$ by SSC during the test, the test piece abruptly comes to rupture when the respective propagated cracks reach a certain specific crack length, at that time, the load that is applied to the test piece is unloaded at the respective cracks that have propagated at almost the same rate when the test piece has ruptured from one certain crack, and the cracks do not propagate any longer in other cracks, it is possible to appropriately calculate the stress intensity factor $K_{ISSC}$ values of the respective remaining initial cracks $11_1$ to $11_{k-1}$ and $11_{k+1}$ to $11_n$.

In addition, according to the method for testing sulfide stress corrosion cracking of a steel material according to the present embodiment, the initial lengths $a_1$ to $a_n$ of the plurality (first to $n^{th}$) of respective initial cracks $11_1$ to $11_n$ introduced by the crack introduction step (step S1) are measured (step S2), and, when the relationship between the initial lengths $a_1$ to $a_n$ of the plurality (first to $n^{th}$) of respective measured initial cracks $11_1$ to $11_n$ and the intervals $d_1$ to $d_{n-1}$ between the initial cracks adjacent to each other $11_1$:$11_2$, ..., $11_{n-1}$:$11_n$ satisfies Expression (2) (when the determination is YES in the step S3), the 4-point bending SSC test step (step S4) is performed.

Therefore, the influence of the stress states at the crack tips of the initial cracks $11_1$ to $11_n$ on other adjacent initial cracks $11_1$ to $11_n$ decreases as much as possible, the adjacent initial cracks $11_1$:$11_2$, ..., $11_{n-1}$:$11_n$ propagate under almost the same conditions, and it is possible to obtain appropriate stress intensity factor $K_{ISSC}$ values.

In addition, according to the method for testing sulfide stress corrosion cracking of a steel material according to the present embodiment, in the stress intensity factor calculation step (step S5), the propagated crack lengths in the sheet thickness direction of the plurality (first to $n^{th}$) of respective initial cracks $11_1$ to $11_n$ in the 4-point bending SSC test step (step S4) are measured (step S51), among the plurality (first to $n^{th}$) of initial cracks $11_1$ to $11_n$, the initial crack in which the crack has propagated the longest in the sheet thickness direction is designated as the any one initial crack $11_k$ ($k^{th}$ among first to $n^{th}$) in the target to be ruptured, and, when the initial length $a_k$ and propagated crack length $b_k$ of the any one initial crack $11_k$ ($k^{th}$ among first to $n^{th}$) in the target to be ruptured satisfy Expression (3) (when the determination is YES in the step 52), the any one initial crack $11_k$ ($k^{th}$ among first to $n^{th}$) in the target to be ruptured is determined to rupture, and the stress intensity factor $K_{ISSC}$ values of the respective remaining initial cracks $11_1$ to $11_{k-1}$ and $11_{k+1}$ to $11_n$ are calculated from Expression (1) (step S53).

Therefore, based on the finding that the calculation of a stress field by the finite element analysis in which the 4-point bending SSC test is reproduced shows that, when the propagated cracks exceed 80% of the sheet thickness t of the test piece 10, the status is the same as when the 4-point bending load does not act on the remaining cracks any longer, it is possible to reliably measure the ruptured initial crack, and it is possible to appropriately calculate the stress intensity factor $K_{ISSC}$ values of the respective remaining initial cracks $11_1$ to $11_{k-1}$ and $11_{k+1}$ to $11_n$.

In addition, according to the method for testing sulfide stress corrosion cracking of a steel material according to the present embodiment, in the stress intensity factor calculation step (step S5), when the stress intensity factor $K_{ISSC}$ values of the respective remaining initial cracks $11_1$ to $11_{k-1}$ and $11_{k+1}$ to $11_n$ have been calculated, in a case where the relationship between the initial lengths $a_1$ to $a_{k-1}$ and $a_{k+1}$ to $a_n$ and propagated crack lengths $b_1$ to $b_{k-1}$ and $b_{k+1}$ to $b_n$ of the respective remaining initial cracks $11_1$ to $11_{k-1}$ and $11_{k+1}$ to $11_n$ and the intervals $d_1$ to $d_{n-1}$ between the adjacent cracks $11_1$:$11_2$, ..., $11_{n-1}$:$11_n$ satisfies Expression (4) (when the determination in the step S54 is YES), the calculated stress intensity factors $K_{ISSC}$ values of the respective remaining initial cracks $11_1$ to $11_{k-1}$ and $11_{k+1}$ to $11_n$ are adopted (step S55).

Therefore, the influence of the stress states at the crack tips of the initial cracks $11_1$ to $11_n$ on other adjacent initial cracks $11_1$ to $11_n$ decreases as much as possible, the adjacent initial cracks $11_1$:$11_2$, ..., $11_{n-1}$:$11_n$ propagate under almost the same conditions, and it is possible to obtain appropriate stress intensity factor $K_{ISSC}$ values.

Although the embodiment of the present invention has been described above, the present invention is not limited thereto and can be modified and improved in various ways.

For example, in the crack introduction step (step S1), it is not always necessary to introduce the plurality of fine initial cracks $11_1$ to $11_n$ into the flat plate-shaped test piece 10 that is collected from the steel material to be evaluated, and one fine initial crack may be introduced.

In addition, in the step S3, at the time of determining whether or not the relationship between the initial lengths $a_1$ to $a_n$ of the plurality (first to $n^{th}$) of respective measured initial cracks $11_1$ to $11_n$ and the intervals $d_1$ to $d_{n-1}$ between the initial cracks adjacent to each other $11_1$:$11_2$, ..., $11_{n-1}$:$11_n$ satisfies Expression (2), it is more preferable to determine whether or not to satisfy Expression (2A) rather than Expression (2).

$$d_1/a_1 \geq 8, \ d_1/a_2 \geq 8, \ d_2/a_2 \geq 8, \ ..., \ d_{n-2}/a_{n-1} \geq 8, \ d_{n-1}/a_{n-1} \geq 8, \ \text{and} \ d_{n-1}/a_n \geq 8 \qquad (2A)$$

In addition, in the step S54, at the time of determining whether or not the relationship between the initial lengths $a_1$ to $a_{k-1}$ and $a_{k+1}$ to $a_n$ and propagated crack lengths $b_1$ to $b_{k-1}$ and $b_{k+1}$ to $b_n$ of the respective remaining initial cracks $11_1$ to $11_{k-1}$ and $11_{k+1}$ to $11_n$ and the intervals $d_1$ to $d_{n-1}$ between the cracks adjacent to each other satisfies Expression (4), it is more preferable to determine whether or not to satisfy Expression (4A) below rather than Expression (4).

$$d_1/(a_1 + b_1) \geq 8,\ d_1/(a_2 + b_2) \geq 8,\ d_2/(a_2 + b_2) \geq 8,\ ...,\ d_{k-2}/(a_{k-1} + b_{k-1}) \geq 8,\ d_{k-1}/(a_{k-1} + b_{k-1}) \geq 8,\ d_k/(a_{k+1} + b_{k+1}) \geq 8,\ d_{k+1}/(a_{k+1} + b_{k+1}) \geq 8,\ ...,\ d_{n-1}/(a_{n-1} + b_{n-1}) \geq 8,\ d_{n-1}/(a_n + b_n) \geq 8 \qquad (4A)$$

In addition, in the step S54, in a case where the relationship between the initial lengths $a_1$ to $a_{k-1}$ and $a_{k+1}$ to $a_n$ and propagated crack lengths $b_1$ to $b_{k-1}$ and $b_{k+1}$ to $b_n$ of the respective remaining initial cracks $11_1$ to $11_{k-1}$ and $11_{k+1}$ to $11_n$ and the intervals $d_1$ to $d_{n-1}$ between the cracks adjacent to each other does not satisfy Expression (4) or Expression (4A), in the step S56, the stress intensity factors $K_{ISSC}$ values of the respective remaining initial cracks $11_1$ to $11_{k-1}$ and $11_{k+1}$ to $11_n$ calculated in the step S53 are not adopted. However, in this case, values obtained by dividing the stress intensity factors $K_{ISSC}$ values of the respective remaining initial cracks $11_1$ to $11_{k-1}$ and $11_{k+1}$ to $11_n$ calculated in the step S53 by a predetermined ratio (for example, 0.8) may be adopted as the stress intensity factors $K_{ISSC}$ values of the respective remaining initial cracks $11_1$ to $11_{k-1}$ and $11_{k+1}$ to $11_n$.

In addition, in the step S52, in a case where the initial length $a_k$ and propagated crack length $b_k$ of the any one initial crack $11_k$ ($k^{th}$ among first to $n^{th}$) in the target to be ruptured do not satisfy Expression (3), the stress intensity factor calculation step (step S5) is ended, and the sulfide stress corrosion cracking test is ended. However, in this case, the step S4 (4-point bending SSC test step) may be continued.

Examples

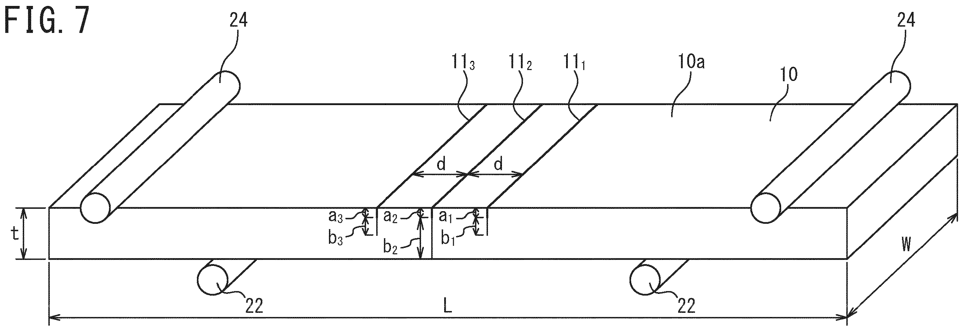

In order to verify the effect of the present invention, an API X65-grade steel pipe having a sheet thickness of 30 mm and an outer diameter of 711 mm was prepared, a test piece 10 having a sheet thickness t of 5 mm, a length L of 125 mm, and a width W of 25 mm was collected from the inner surface of the steel pipe, and three fine initial cracks $11_1$ to $11_3$ were introduced into a surface 10a (a surface matching the inner surface of the steel pipe) of the test piece 10 with a short-pulse laser microprocessing machine having a laser type of SHG-YAG (second-harmonic, green laser), a pulse width of 8 picoseconds, and an output of 30 KW (refer to FIG. 7). As illustrated in FIG. 7, the three respective introduced fine initial cracks $11_1$ to $11_3$ were processed at pitches d of 10 mm inside between 4-point bending load jigs 22 across a short span in dimensions of initial lengths $a_1$ to $a_3$ of 120 $\mu$m and a width w of 30 $\mu$m (Expression (2) was satisfied). A 4-point bending SSC test was performed by immersing the test piece 10 in which the three fine initial cracks $11_1$ to $11_3$ were processed in a test solution in a 4-point bent state. This 4-point bending SSC test was performed according to NACE TM0316-2016. The conditions of the 4-point bending SSC test are as follows.

Test solution: NACE A Solution
Load (bending) stress: 465 MPa (90% of the yield stress of the material)
Test environment: pH 2.6 to pH 4.0
Test temperature: 24°C $\pm$ 3°C
Hydrogen sulfide partial pressure: 1 bar
Specific liquid volume: 30 $\pm$ 10 ml/m$^2$
Test period: 720 hours (30 days)

In addition, after 30 days of the 4-point bending SSC test, the test piece 10 was taken out, and propagated crack lengths from the respective initial cracks $11_1$ to $11_3$ (initial lengths $a_1$ to $a_3$ + propagated crack lengths $b_1$ to $b_3$) were measured. The initial crack in which the crack had propagated the longest was the initial crack $11_2$ at the center of the test piece 10 in the longitudinal direction, the total of the initial length $a_2$ and the propagated crack length $b_2$ was 4900 $\mu$m, and the rupture condition of Expression (3) was satisfied. Regarding the dimensions of the remaining initial cracks $11_1$ and $11_3$, the totals of the initial length $a_1$ or $a_3$ and the propagated crack length $b_1$ or $b_3$ were 1300 $\mu$m and 1320 $\mu$m, respectively, and both were almost the same cracks. When the stress intensity factors $K_{ISSC1}$ value was obtained from Expression (1) based on the total of the initial length $a_1$ and propagated crack length $b_1$ of this remaining initial crack $11_1$ of 1300 $\mu$m and the initial load (bending) stress $\sigma$ of 465 MPa, the stress intensity factors $K_{ISSC1}$ value was as follows.

[Math. 2]

$$33\,\text{MPa}\sqrt{m}$$

In addition, similarly, the stress intensity factors $K_{ISSC3}$ value obtained from Expression (1) based on the total of the initial length $a_3$ and propagated crack length $b_3$ of the remaining initial crack $11_3$ of 1320 $\mu$m and the initial load (bending) stress $\sigma$ of 465 MPa was also the same.

The relationship between the initial lengths $a_1$ and $a_3$ and propagated crack lengths $b_1$ and $b_3$ of the respective

remaining initial cracks $11_1$ and $11_3$ and the intervals d = 10 mm between the adjacent cracks $11_1$:$11_2$ and $11_2$:$11_3$ satisfied Expression (4).

As described above, it was possible to obtain the stress intensity factor $K_{ISSC}$ value in the sheet thickness direction of the steel material to be evaluated (steel pipe).

Reference Signs List

| 10 | test piece |
|---|---|
| 10a | surface |
| $11_1$ to $11_n$ | initial crack |
| 20 | 4-point bending ssc test jig |
| 21 | pedestal |
| 22 | 4-point bending load jig |
| 23 | support member |
| 24 | 4-point bending load jig |
| $a_1$ to $a_n$ | initial length |
| $b_1$ to $b_n$ | propagated crack length |

**Claims**

1. A method for testing sulfide stress corrosion cracking of a steel material, comprising:

a crack introduction step of introducing, into a test piece (10) that is collected from a steel material to be evaluated, a fine initial crack (11) that extends in a sheet thickness direction from a surface (10a) of the test piece (10) and is 2 $\mu$m to 1000 $\mu$m in width and 1 $\mu$m to 1000 $\mu$m in initial length;

a 4-point bending sulfide stress cracking (SSC) test step of performing a 4-point bending SSC test in which the test piece (10) into which the initial crack (11) has been introduced in the crack introduction step is immersed in a test solution in a 4-point bent state; and

a stress intensity factor calculation step of calculating a stress intensity factor $K_{ISSC}$ value based on a state where the initial crack (11) has propagated in the sheet thickness direction by the 4-point bending SSC test step, wherein, in the crack introduction step, a plurality (first to $n^{th}$, n = 2 or more) of the initial cracks (11) that extends in the sheet thickness direction from the surface (10a) of the test piece (10) is introduced along a row direction, in the 4-point bending SSC test step, the test piece (10) into which the plurality, that is first to $n^{th}$, of the initial cracks (11) has been introduced is immersed in the test solution in a 4-point bent state, in the stress intensity factor calculation step, the stress intensity factors $K_{ISSC}$ values of the respective remaining initial cracks (11) are calculated from Expression (1) below based on the initial lengths and propagated crack lengths of a plurality, that is first to $k-1^{th}$ and $k+1^{th}$ to $n^{th}$, of remaining cracks in a sample in which any one initial crack (11), that is $k^{th}$ among first to $n^{th}$, among the plurality, that is first to $n^{th}$, of the initial cracks (11) has been ruptured due to propagation in the sheet thickness direction by the 4-point bending SSC test step,

[Math. 1]

$$K_{ISSC1} = F \cdot \sigma \sqrt{\pi (a_1 + b_1)} \ , \ K_{ISSC2} = F \cdot \sigma \sqrt{\pi (a_2 + b_2)} \ , \cdots ,$$

$$K_{ISSCk-1} = F \cdot \sigma \sqrt{\pi (a_{k-1} + b_{k-1})} \ , \ K_{ISSCk+1} = F \cdot \sigma \sqrt{\pi (a_{k+1} + b_{k+1})} \ , \cdots ,$$

$$K_{ISSCn} = F \cdot \sigma \sqrt{\pi (a_n + b_n)} \quad \cdots \quad (1)$$

here, $K_{ISSC1}$ to $K_{ISSCk-1}$ and $K_{ISSCk+1}$ to $K_{ISSCn}$ are stress intensity factor $K_{ISSC}$ values of the respective remaining initial cracks (11), F is a shape factor, $\sigma$ is a bending stress that is applied to the test piece (10) by the 4-point bending SSC test step, $a_1$ to $a_{k-1}$ and $a_{k+1}$ to $a_n$ are measured initial lengths of the respective remaining initial cracks (11), and $b_1$ to $b_{k-1}$ and $b_{k+1}$ to $b_n$ are measured propagated crack lengths of the respective remaining initial cracks (11).

2. The method for testing sulfide stress corrosion cracking of a steel material according to claim 1, wherein, the initial lengths of the plurality, that is first to $n^{th}$, of the respective initial cracks (11) introduced by the crack introduction step are measured, and, when a relationship between the initial lengths of the plurality, that is first to $n^{th}$, of the respective measured initial cracks (11) and intervals between the initial cracks (11) adjacent to each other satisfies Expression (2), the 4-point bending SSC test step is performed,

$$d_1/a_1 \geq 5, \ d_1/a_2 \geq 5, \ d_2/a_2 \geq 5, \ ..., \ d_{n-2}/a_{n-1} \geq 5, \ d_{n-1}/a_{n-1} \geq 5, \ \text{and} \ d_{n-1}/a_n \geq 5 \ ... \ (2)$$

here, $a_1$ to $a_n$ are the initial lengths of the plurality, that is first to $n^{th}$, of the respective initial cracks (11), and $d_1$ to $d_{n-1}$ are the intervals between the initial cracks (11) adjacent to each other.

3. The method for testing sulfide stress corrosion cracking of a steel material according to claim 2, wherein, in the stress intensity factor calculation step, the propagated crack lengths in the sheet thickness direction of the plurality, that is first to $n^{th}$, of the respective initial cracks (11) in the 4-point bending SSC test step are measured, among the plurality, that is first to $n^{th}$, of initial cracks (11), an initial crack (11) in which the crack has propagated longest in the sheet thickness direction is designated as the any one initial crack (11), that is $k^{th}$ among first to $n^{th}$, in the target to be ruptured, and, when the initial length and propagated crack length of the any one initial crack (11), that is $k^{th}$ among first to $n^{th}$, in the target to be ruptured satisfy Expression (3), the any one initial crack (11), that is $k^{th}$ among first to $n^{th}$, in the target to be ruptured is determined to rupture, and the stress intensity factor $K_{ISSC}$ values of the respective remaining initial cracks (11) are calculated from Expression (1),

$$0.8t \leq (a_k + b_k) \leq 1.0t \ ... \ (3)$$

here, $a_k$ is the measured initial length of the any one, that is $k^{th}$ among first to $n^{th}$, of initial crack (11) in the target to be ruptured, $b_k$ is the measured propagated crack length of the any one, that is $k^{th}$ among first to $n^{th}$, of initial crack (11) in the target to be ruptured, and t is a sheet thickness of a test piece (10).

4. The method for testing sulfide stress corrosion cracking of a steel material according to claim 3, wherein, in the stress intensity factor calculation step, when the stress intensity factor $K_{ISSC}$ values of the respective remaining initial cracks (11) have been calculated, the calculated stress intensity factors $K_{ISSC}$ values of the respective remaining initial cracks (11) are adopted in a case where the relationship between the initial lengths and propagated crack lengths of the respective remaining initial cracks (11) and the intervals between the adjacent cracks satisfies Expression (4) below,

$$d_1/(a_1 + b_1) \geq 5, \ d_1/(a_2 + b_2) \geq 5, \ d_2/(a_2 + b_2) \geq 5, \ ..., \ d_{k-2}/(a_{k-1} + b_{k-1}) \geq 5, \ d_{k-1}(a_{k-1} + b_{k-1}) \geq 5, \ d_k(a_{k+1} + b_{k+1}) \geq 5, \ d_{k+1}/(a_{k+1} + b_{k+1}) \geq 5, \ ..., \ d_{n-1}/(a_{n-1} + b_{n-1}) \geq 5, \ d_{n-1}/(a_n + b_n) \geq 5 \quad (4)$$

here, $a_1$ to $a_{k-1}$ and $a_{k+1}$ to $a_n$ are the measured initial lengths of the respective remaining initial cracks (11), that is first to $k-1^{th}$ and $k+1^{th}$ to $n^{th}$, $b_1$ to $b_{k-1}$ and $b_{k+1}$ to $b_n$ are the measured propagated crack lengths of the respective remaining initial cracks (11), that is first to $k-1^{th}$ and $k+1^{th}$ to $n^{th}$, and $d_1$ to $d_{n-1}$ are the intervals between the cracks adjacent to each other.

**Patentansprüche**

1. Verfahren zum Prüfen von sulfidinduzierter Spannungsrisskorrosion eines Stahlmaterials, welches umfasst:

einen Riss-Einführungsschritt des Einbringens eines feinen Anfangsrisses (11) in ein Prüfstück (10), das aus einem zu bewertenden Stahlmaterial entnommen wurde, welcher sich in einer Blechdickenrichtung von einer Oberfläche (10a) des Prüfstücks (10) aus erstreckt und eine Breite von 2 µm bis 1000 µm und eine Anfangslänge von 1 µm bis 1000 µm aufweist,
einen 4-Punkt-Biege-Prüfschritt der Sulfidspannungsrissbildung (SSC) des Durchführens einer 4-Punkt-Biege-SSC-Prüfung, bei der das Prüfstück (10), in das im Riss-Einführungsschritt der Anfangsriss (11) eingeführt wurde, in einem 4-Punkt-Biegezustand in eine Prüflösung eingetaucht wird, und
einen Spannungsintensitätsfaktor-Berechnungsschritt des Berechnens eines $K_{ISCC}$-Wertes für den Spannungsintensitätsfaktor auf der Grundlage eines Zustands, in dem sich der Anfangsriss (11) durch den 4-Punkt-Biege-SSC-Prüfschritt in Richtung der Blechdicke ausgebreitet hat, wobei im Riss-Einführungsschritt eine Vielzahl (erster bis $n^{ter}$, n = 2 oder mehr) der Anfangsrisse (11), welche sich in Richtung der Blechdicke von der Oberfläche (10a) des Prüfstücks (10) in Richtung der Blechdicke erstreckt, entlang einer Reihenrichtung eingebracht wird, im 4-Punkt-Biege-SSC-Prüfschritt das Prüfstück (10), in das die Vielzahl, das heißt die ersten bis $n^{ten}$, der Anfangsrisse (11) eingebracht wurde, in einem 4-Punkt-Biegezustand in die Prüflösung getaucht wird, im Schritt zur Berechnung des Spannungsintensitätsfaktors die $K_{ISCC}$-Werte der Spannungsintensitätsfaktoren der jeweiligen verbleibenden Anfangsrisse (11) aus dem nachstehenden Ausdruck (1) auf der Grundlage der Anfangslängen und der sich ausbreitet habenden Risslängen einer Vielzahl, das heißt des ersten bis zum $k-1^{ten}$ und des $k+1^{ten}$ bis zum $n^{ten}$, der verbleibenden Risse in einer Probe berechnet werden, in welcher ein beliebiger Anfangsriss (11), das heißt der $k^{te}$ unter dem ersten bis $n^{ten}$, unter der Vielzahl, das heißt dem ersten bis $n^{ten}$, der Anfangsrisse (11) aufgrund der Ausbreitung in der Blechdickenrichtung durch den 4-Punkt-Biege-SSC-Prüfschritt gebrochen ist,

[Math. 1]

$$K_{ISCC1}=F \cdot \sigma \sqrt{\pi(a_1+b_1)} \quad , \quad K_{ISCC2}=F \cdot \sigma \sqrt{\pi(a_2+b_2)} \quad , \cdots ,$$

$$K_{ISCCk-1}=F \cdot \sigma \sqrt{\pi(a_{k-1}+b_{k-1})} \quad , \quad K_{ISCCk-1}=F \cdot \sigma \sqrt{\pi(a_{k+1}+b_{k+1})} \quad , \cdots ,$$

$$K_{ISCCn}=F \cdot \sigma \sqrt{\pi(a_n+b_n)} \quad \cdots \quad (1)$$

Hier sind $K_{ISCC1}$ bis $K_{ISCCk-1}$ und $K_{ISCCk+1}$ bis $K_{ISCCn}$ die $K_{ISCC}$-Werte des Spannungsintensitätsfaktors der jeweiligen verbleibenden Anfangsrisse (11), F ist ein Formfaktor, $\sigma$ ist eine Biegespannung, die durch den 4-Punkt-Biege-SSC-Prüfschritt auf das Prüfstück (10) ausgeübt wird, $a_1$ bis $a_{k-1}$ und $a_{k+1}$ bis $a_n$ sind gemessene Anfangslängen der jeweiligen verbleibenden Anfangsrisse (11), und $b_1$ bis $b_{k-1}$ und $b_{k+1}$ bis $b_n$ sind gemessene fortgeschrittene Risslängen der jeweiligen verbleibenden Anfangsrisse (11).

2. Verfahren zum Prüfen von sulfidinduzierter Spannungsrisskorrosion eines Stahlmaterials gemäß Anspruch 1, wobei die Anfangslängen der Vielzahl, das heißt des ersten bis $n^{ten}$, der jeweiligen durch den Riss-Einführungsschritt eingeführten Anfangsrisse (11) gemessen werden, und, wenn eine Beziehung zwischen den Anfangslängen der Vielzahl, das heißt des ersten bis $n^{ten}$, der jeweiligen gemessenen Anfangsrisse (11) und den Abständen zwischen den benachbarten Anfangsrissen (11) den Ausdruck (2) erfüllt, der 4-Punkt-Biege-SSC-Prüfschritt durchgeführt wird,

$$d_1/a_1 \geq 5, \, d_1/a_2 \geq 5, \, d_2/a_2 \geq 5, \, ..., \, d_{n-2}/a_{n-1} \geq 5, \, d_{n-1}/a_{n-1} \geq 5 \text{ und } d_{n-1}/a_n \geq 5 \, ... \, (2)$$

Hier sind $a_1$ bis $a_n$ die Anfangslängen der Vielzahl, das heißt des ersten bis zur $n^{ten}$, der jeweiligen Anfangsrisse (11) und $d_1$ bis $d_{n-1}$ sind die Abstände zwischen den benachbarten Anfangsrissen (11).

3. Verfahren zum Prüfen von sulfidinduzierter Spannungsrisskorrosion eines Stahlmaterials gemäß Anspruch 2, wobei im Schritt zur Berechnung des Spannungsintensitätsfaktors die fortgeschrittenen Risslängen in Blechdickenrichtung der Vielzahl, das heißt des ersten bis $n^{ten}$, der jeweiligen Anfangsrisse (11) im 4-Punkt-Biege-SSC-Prüfschritt gemessen werden, unter der Vielzahl, das heißt dem ersten bis $n^{ten}$, der Anfangsrisse (11) ein Anfangsriss (11), bei dem sich der Riss in Richtung der Blechdicke am weitesten ausgebreitet hat, als der eine beliebige Anfangsriss (11), das heißt der $k^{te}$ unter dem ersten bis $n^{ten}$, im zu brechenden Zielobjekt bezeichnet wird, und, wenn die Anfangslänge und die ausgeweitete Risslänge des einen beliebigen Anfangsrisses (11), das heißt des $k^{ten}$ unter dem ersten bis $n^{ten}$, im zu brechenden Zielobjekt den Ausdruck (3) erfüllen, der eine Anfangsriss (11), das heißt der $k^{te}$ unter dem ersten bis zum $n^{ten}$, im zu brechenden Zielobjekt zum Brechen bestimmt wird, und die $K_{ISCC}$-Werte des Spannungsintensitätsfaktors der jeweiligen verbleibenden Anfangsrisse (11) aus Ausdruck (1) berechnet werden,

$$0{,}8t \leq (a_k + b_k) \leq 1{,}0t \, ... \, (3)$$

Hier ist $a_k$ die gemessene Anfangslänge des einen beliebigen, das heißt des $k^{ten}$ unter dem ersten bis $n^{ten}$, Anfangsrisses (11) im zu brechenden Zielobjekt, $b_k$ ist die gemessene fortgeschrittene Risslänge des einen beliebigen, das heißt des $k^{ten}$ unter dem ersten bis $n^{ten}$, Anfangsrisses (11) im zu brechenden Zielobjekt ist und t ist die Blechdicke eines Prüfstücks (10).

4. Verfahren zum Prüfen von sulfidinduzierter Spannungsrisskorrosion eines Stahlmaterials gemäß Anspruch 3, wobei im Schritt zur Berechnung des Spannungsintensitätsfaktors, wenn die $K_{ISCC}$-Werte des Spannungsintensitätsfaktors der jeweiligen verbleibenden Anfangsrisse (11) berechnet worden sind, die berechneten $K_{ISCC}$-Werte des Spannungsintensitätsfaktors der jeweiligen verbleibenden Anfangsrisse (11) übernommen werden, sofern die Beziehung zwischen den Anfangslängen und den fortgeschrittenen Risslängen der jeweiligen verbleibenden Anfangsrisse (11) und die Abstände zwischen den benachbarten Rissen den nachstehenden Ausdruck (4) erfüllt:

$$d_1/(a_1 + b_1) \geq 5,\ d_1/(a_2 + b_2) \geq 5,\ d_2/(a_2 + b_2) \geq 5,\ ...,\ d_{k-2}/(a_{k-1} + b_{k-1}) \geq 5,\ d_{k-1}/(a_{k-1} + b_{k-1}) \geq 5,\ d_k/(a_{k+1} + b_{k+1}) \geq 5,\ d_{k+1}/(a_{k+1} + b_{k+1}) \geq 5,\ ...,\ d_{n-1}/(a_{n-1} + b_{n-1}) \geq 5,\ d_{n-1}/(a_n + b_n) \geq 5 \tag{4}$$

Hier sind $a_1$ bis $a_{k-1}$ und $a_{k+1}$ bis $a_n$ die gemessenen Anfangslängen der jeweiligen verbleibenden Anfangsrisse (11), das heißt des ersten bis zum k-1$^{\text{ten}}$ und des k+1$^{\text{ten}}$ bis zum n$^{\text{ten}}$, $b_1$ bis $b_{k-1}$ und $b_{k+1}$ bis $b_n$ sind die gemessenen fortgeschrittenen Risslängen der jeweiligen verbleibenden Anfangsrisse (11), das heißt des ersten bis zum k-1$^{\text{ten}}$ und des k+1$^{\text{ten}}$ bis zum n$^{\text{ten}}$, und $d_1$ bis $d_{n-1}$ sind die Abstände zwischen den benachbarten Rissen.

## Revendications

1. Procédé d'essai de fissuration par corrosion sous contrainte due aux sulfures d'un matériau en acier, comprenant :

   une étape d'introduction de fissure consistant à introduire, dans une pièce d'essai (10) qui est prélevée dans un matériau en acier à évaluer, une fissure initiale fine (11) qui s'étend dans une direction d'épaisseur de tôle depuis une surface (10a) de la pièce d'essai (10), et ayant une largeur de 2 $\mu$m à 1 000 $\mu$m et une longueur initiale de 1 $\mu$m à 1 000 $\mu$m ;
   une étape d'essai de fissuration sous contrainte due aux sulfures (SSC) en flexion en 4 points consistant à effectuer un essai SSC en flexion en 4 points au cours de laquelle la pièce d'essai (10), dans laquelle la fissure initiale (11) a été introduite lors de l'étape d'introduction de fissure, est immergée dans une solution d'essai dans un état fléchi en 4 points ; et
   une étape de calcul de facteur d'intensité de contrainte consistant à calculer une valeur $K_{ISSC}$ de facteur d'intensité de contrainte sur la base d'un état où la fissure initiale (11) s'est propagée dans la direction d'épaisseur de tôle par l'étape d'essai SSC en flexion en 4 points, dans lequel, lors de l'étape d'introduction de fissure, une pluralité (première à n$^{\text{ème}}$, n = 2 ou plus) des fissures initiales (11) qui s'étendent dans la direction d'épaisseur de tôle depuis la surface (10a) de la pièce d'essai (10), est introduite le long d'une direction de rangée, lors de l'étape d'essai SSC en flexion en 4 points, la pièce d'essai (10) dans laquelle la pluralité, c'est-à-dire première à n$^{\text{ème}}$, des fissures initiales (11) a été introduite est immergée dans la solution d'essai dans un état fléchi en 4 points, lors de l'étape de calcul de facteur d'intensité de contrainte, les valeurs $K_{ISSC}$ de facteurs d'intensité de contrainte des fissures initiales (11) restantes respectives sont calculées d'après l'Expression (1) ci-dessous, sur la base des longueurs initiales et des longueurs de fissure propagée d'une pluralité, c'est-à-dire première à k-1$^{\text{ème}}$ et k+1$^{\text{ème}}$ à n$^{\text{ème}}$, de fissures restantes dans un échantillon dans lequel une quelconque fissure initiale (11), c'est-à-dire la k$^{\text{ème}}$ parmi les première à n$^{\text{ème}}$, parmi la pluralité, c'est-à-dire première à n$^{\text{ème}}$, des fissures initiales (11) a été rompue en raison de la propagation dans la direction d'épaisseur de tôle par l'étape d'essai SSC en flexion en 4 points,
   [Math. 1]

   $$K_{ISSC1} = F \cdot \sigma \sqrt{\pi(a_1 + b_1)}\ ,\ K_{ISSC2} = F \cdot \sigma \sqrt{\pi(a_2 + b_2)}\ ,\ \cdots,$$

   $$K_{ISSCk-1} = F \cdot \sigma \sqrt{\pi(a_{k-1} + b_{k-1})}\ ,\ K_{ISSCk+1} = F \cdot \sigma \sqrt{\pi(a_{k+1} + b_{k+1})}\ ,\ \cdots,$$

   $$K_{ISSCn} = F \cdot \sigma \sqrt{\pi(a_n + b_n)}\ \cdots\ (1)$$

   ici, $K_{ISSC_1}$ à $K_{ISSC_{k-1}}$ et $K_{ISSC_{k+1}}$ à $K_{ISSC_n}$ sont des valeurs $K_{ISSC}$ de facteur d'intensité de contrainte des fissures initiales (11) restantes respectives, F est un facteur de forme, $\sigma$ est une contrainte de flexion qui est appliquée à la pièce d'essai (10) par l'étape d'essai SSC en flexion en 4 points, $a_1$ à $a_{k-1}$ et $a_{k+1}$ à $a_n$ sont des longueurs initiales mesurées des fissures initiales (11) restantes respectives, et $b_1$ à $b_{k-1}$ et $b_{k+1}$ à $b_n$ sont des longueurs de fissure propagée mesurées des fissures initiales (11) restantes respectives.

2. Procédé d'essai de fissuration par corrosion sous contrainte due aux sulfures d'un matériau en acier selon la revendication 1, dans lequel, les longueurs initiales de la pluralité, c'est-à-dire première à n$^{\text{ème}}$, des fissures initiales (11) respectives introduites par l'étape d'introduction de fissure sont mesurées, et, lorsqu'une relation entre les longueurs initiales de la pluralité, c'est-à-dire première à n$^{\text{ème}}$, des fissures initiales (11) mesurées respectives et des intervalles entre les fissures initiales (11) mutuellement adjacentes satisfait à l'Expression (2), l'étape d'essai SSC en flexion en 4 points est effectuée,

$$d_1/a_1 \geq 5, \; d_1/a_2 \geq 5, \; d_2/a_2 \geq 5, \; ..., \; d_{n-2}/a_{n-1} \geq 5, \; d_{n-1}/a_{n-1} \geq 5, \; \text{et } d_{n-1}/a_n \geq 5 \; ... \; (2)$$

ici, $a_1$ à $a_n$ sont les longueurs initiales de la pluralité, c'est-à-dire première à $n^{\text{ème}}$, des fissures initiales (11) respectives, et $d_1$ à $d_{n-1}$ sont les intervalles entre les fissures initiales (11) mutuellement adjacentes.

3. Procédé d'essai de fissuration par corrosion sous contrainte due aux sulfures d'un matériau en acier selon la revendication 2, dans lequel, lors de l'étape de calcul de facteur d'intensité de contrainte, les longueurs de fissure propagée dans la direction d'épaisseur de tôle de la pluralité, c'est-à-dire première à $n^{\text{ème}}$, des fissures initiales (11) respectives lors de l'étape d'essai SSC en flexion en 4 points sont mesurées, parmi la pluralité, c'est-à-dire première à $n^{\text{ème}}$, de fissures initiales (11), une fissure initiale (11) dans laquelle la fissure s'est propagée sur la plus grand longueur dans la direction d'épaisseur de tôle est désignée comme la quelconque fissure initiale (11), c'est-à-dire la $k^{\text{ème}}$ parmi les première à $n^{\text{ème}}$, dans la cible à rompre, et, lorsque la longueur initiale et la longueur de fissure propagée de la quelconque fissure initiale (11), c'est-à-dire la $k^{\text{ème}}$ parmi les première à $n^{\text{ème}}$, dans la cible à rompre satisfont à l'Expression (3), la quelconque fissure initiale (11), c'est-à-dire la $k^{\text{ème}}$ parmi les première à $n^{\text{ème}}$, dans la cible à rompre est déterminée comme étant rompue, et les valeurs KISSC de facteur d'intensité de contrainte des fissures initiales (11) restantes respectives sont calculées d'après l'Expression (1),

$$0{,}8t \leq (a_k + b_k) \leq 1{,}0t \; ... \; (3)$$

ici, $a_k$ est la longueur initiale mesurée de la quelconque, c'est-à-dire la $k^{\text{ème}}$ parmi les première à $n^{\text{ème}}$, de fissure initiale (11) dans la cible à rompre, $b_k$ est la longueur de fissure propagée mesurée de la quelconque, c'est-à-dire la $k^{\text{ème}}$ parmi les première à $n^{\text{ème}}$, de fissure initiale (11) dans la cible à rompre, et $t$ est une épaisseur de tôle de la pièce d'essai (10).

4. Procédé d'essai de fissuration par corrosion sous contrainte due aux sulfures d'un matériau en acier selon la revendication 3, dans lequel, lors de l'étape de calcul de facteur d'intensité de contrainte, lorsque les valeurs $K_{ISSC}$ de facteur d'intensité de contrainte des fissures initiales (11) restantes respectives ont été calculées, les valeurs $K_{ISSC}$ de facteurs d'intensité de contrainte calculées des fissures initiales (11) restantes respectives sont adoptées dans le cas où la relation entre les longueurs initiales et les longueurs de fissure propagée des fissures initiales (11) restantes respectives et les intervalles entre les fissures adjacentes satisfait à l'Expression (4) ci-dessous,

$$d_1/(a_1 + b_1) \geq 5, \; d_1/(a_2 + b_2) \geq 5, \; d_2/(a_2 + b_2) \geq 5, \; ..., \; d_{k-2}/(a_{k-1} + b_{k-1}) \geq 5, \; d_{k-1}/(a_{k-1} + b_{k-1}) \geq 5, \; d_k/(a_{k+1} + b_{k+1}) \geq 5, \; d_{k+1}/(a_{k+1} + b_{k+1}) \geq 5, \; ..., \; d_{n-1}/(a_{n-1} + b_{n-1}) \geq 5, \; d_{n-1}/(a_n + b_n) \geq 5 \quad (4)$$

ici, $a_1$ à $a_{k-1}$ et $a_{k+1}$ à $a_n$ sont les longueurs initiales mesurées des fissures initiales (11) restantes respectives, c'est-à-dire de la première à la $k-1^{\text{ème}}$ et de la $k+1^{\text{ème}}$ à la $n^{\text{ème}}$, $b_1$ à $b_{k-1}$ et $b_{k+1}$ à $b_n$ sont les longueurs de fissure propagée mesurées des fissures initiales (11) restantes respectives, c'est-à-dire de la première à la $k-1^{\text{ème}}$ et de la $k+1^{\text{ème}}$ à la $n^{\text{ème}}$, et $d_1$ à $d_{n-1}$ sont les intervalles entre les fissures mutuellement adjacentes.

# FIG. 1

START

S1 — INTRODUCTION OF INITIAL CRACKS

S2 — MEASUREMENT OF INITIAL LENGTH
OF EACH INITIAL CRACK

S3 — RELATIONSHIP
BETWEEN INITIAL LENGTH
OF EACH INITIAL CRACK AND ADJACENT
INITIAL CRACK INTERVAL SATISFIES
EXPRESSION (2) ?

N

Y

S4 — PERFORMING OF 4-POINT BENDING
SSC TEST

S5 — CALCULATION OF STRESS
INTENSITY FACTOR $K_{ISSC}$ VALUE

END

EP 4 276 442 B1

# FIG. 2

START

S51 — MEASUREMENT OF PROPAGATED CRACK LENGTH IN SHEET THICKNESS DIRECTION OF EACH INITIAL CRACK

S52 — INITIAL LENGTH AND PROPAGATED CRACK LENGTH OF INITIAL CRACK IN WHICH CRACK HAS PROPAGATED LONGEST SATISFY EXPRESSION (3)? — N

Y

S53 — INITIAL CRACK IS DETERMINED TO RUPTURE, STRESS INTENSITY FACTOR $K_{ISSC}$ VALUE OF EACH REMAINING INITIAL CRACK IS CALCULATED FROM EXPRESSION (1)

S54 — RELATIONSHIP BETWEEN INITIAL LENGTH AND PROPAGATED CRACK LENGTH OF EACH REMAINING INITIAL CRACK AND ADJACENT CRACK INTERVAL SATISFIES EXPRESSION (4)? — N

Y

S55 — CALCULATED STRESS INTENSITY FACTOR $K_{ISSC}$ VALUE IS ADOPTED

S56 — CALCULATED STRESS INTENSITY FACTOR $K_{ISSC}$ VALUE IS NOT ADOPTED

END

15

FIG. 3

EP 4 276 442 B1

# FIG. 4

# FIG. 5

# FIG. 6

FIG. 7

**EP 4 276 442 B1**

**Patent documents cited in the description**

- JP 2008051632 B